(19) Europäisches Patentamt
European Patent Office
Office européen des brevets

(11) EP 2 907 518 A1

(12) **EUROPÄISCHE PATENTANMELDUNG**

(43) Veröffentlichungstag:
19.08.2015  Patentblatt 2015/34

(51) Int Cl.:
*A61K 31/724* (2006.01)     *A61K 9/08* (2006.01)
*A61K 9/00* (2006.01)       *A61P 29/00* (2006.01)

(21) Anmeldenummer: 14155234.9

(22) Anmeldetag: 14.02.2014

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(71) Anmelder: SapioTec GmbH
97082 Würzburg (DE)

(72) Erfinder:
• Roewer, Norbert
  97082 Würzburg (DE)
• Broscheit, Jens
  97209 Würzburg (DE)

(74) Vertreter: Glawe, Delfs, Moll
Partnerschaft mbB von
Patent- und Rechtsanwälten
Rothenbaumchaussee 58
20148 Hamburg (DE)

(54) **Analgetikum**

(57)    Die Erfindung betrifft eine pharmazeutische Zusammensetzung enthaltend methyliertes β-Cyclodextrin zur Verwendung als Analgetikum und/oder Antiphlogistikum. Die Erfindung hat erkannt, dass auf diese Weise nociceptives PGE2 in vivo wirksam komplexiert und damit gebunden werden kann.

Fig. 1

**Beschreibung**

Die Erfindung betrifft eine pharmazeutische Zusammensetzung zur Verwendung als Analgetikum und/oder Antiphlogistikum.

Analgetika und Antiphlogistika sind aus offenkundiger Vorbenutzung vielfach bekannt.

Der vorliegenden Erfindung liegt die Aufgabe zu Grunde, eine pharmazeutische Zusammensetzung zu schaffen, die sich zur Verwendung als Antiphlogistikum und insbesondere Analgetikum eignet.

Gelöst wird diese Aufgabe durch eine pharmazeutische Zusammensetzung gemäß dem Hauptanspruch. Die Erfindung betrifft somit insbesondere eine pharmazeutische Zusammensetzung enthaltend methyliertes $\beta$-Cyclodextrin zur Verwendung als Analgetikum und/oder Antiphlogistikum.

Zunächst seien einige im Rahmen der Erfindung verwendete Begriffe erläutert.

Cyclodextrine sind zyklische Oligosaccharide aus $\alpha$-1,4-glykosidisch verknüpften Glukosemolekülen. $\beta$-Cyclodextrin besitzt sieben Glukoseeinheiten. Bei einem methylierten ß-Cyclodextrin sind Hydroxygruppen der Glukoseeinheit mit Methylgruppen versehen. Erfindungsgemäß ist in der Regel lediglich ein Teil der 21 Hydroxygruppen eines ß-Cyclodextrins methyliert.

Die Herstellung von methylierten ß-Cyclodextrinen ist dem Fachmann geläufig, entsprechende Produkte sind bspw. von der Wacker Chemie unter der Bezeichnung Cavasol® erhältlich.

Antiphlogistika sind oder enthalten entzündungshemmende Wirkstoffe und greifen in der Regel auf biochemischen Weg in den Entzündungsprozess ein.

Analgetika vermindern das Schmerzempfinden. Eine Untergruppe der Analgetika sind die Antineoceptiva, die die Reizschwelle der Nociceptoren des Nervensystems herabsetzen und damit das Schmerzempfinden vermindern. Im Rahmen der Erfindung ist eine pharmazeutische Zusammensetzung zur Verwendung als Analgetikum und insbesondere Antineoceptivum besonders bevorzugt.

Bevorzugt wird als methyliertes $\beta$-Cyclodextrin RAMEB (randomly methylated $\beta$-Cyclodextrin) eingesetzt. Es handelt sich um ein statistisch methyliertes $\beta$-Cyclodextrin mit einem Substitutionsgrad von etwa 1,8 Methylgruppen pro Zuckereinheit bzw. 12,5 Methylgruppen pro Cyclodextrinring (DS degree of substitution ungefähr 12,5). RAMEB ist kommerziell erhältlich bspw. von Wacker Chemie unter der Bezeichnung Cavasol® W7 M Pharma.

Die Erfindung hat gemäß einer besonders vorteilhaften Ausführungsform erkannt, dass sich methyliertes $\beta$-Cyclodextrin mit einem durchschnittlichen Substitutionsgrad von etwa 9 bis 12, vorzugsweise 10 bis 12, weiter vorzugsweise 10 bis 11 Methylgruppen pro Cyclodextrinring in besonderem Maße für die erfindungsgemäß vorgesehene Verwendung eignet. Es kann gemäß einem unten näher beschriebenen weiteren Aspekt der Erfindung beispielsweise hergestellt werden durch chromatografische Fraktionierung von RAMEB. Der Einsatz von kommerziell erhältlichem RAMEB ist gemäß einem weiteren nicht bindenden Erklärungsversuch unter Umständen deswegen möglich, weil dieses kommerziell erhältliche Produkt aufgrund der statistischen Verteilung einen hinreichenden Anteil von methylierten $\beta$-Cyclodextrinen mit dem beschriebenen besonders bevorzugten Bereich des Substitutionsgrades enthält.

Ein den Schutzbereich nicht beschränkender und die Anmelderin nicht bindender Erklärungsversuch für die Wirkungsweise der Erfindung ist, dass die erfindungsgemäße Zusammensetzung in der Lage ist, in vivo PGE2 (Prostaglandin E2) komplexiert. PGE2 ist ein Hauptprostaglandin, welches in das Entzündungsgeschehen involviert ist. Es erhöht die Gefäßpermeabilität (Gewebeschwellung), ist an der Entstehung der Rötung beteiligt und verstärkt den Schmerz, indem es nozizeptive Nervenendigungen sensibilisiert.

Die Erfindung hat erkannt, dass insbesondere methylierte $\beta$-Cyclodextrine mit den beschriebenen bevorzugten Bereichen des Substitutionsgrads einen besonders ausgewogenen Kompromiss hinsichtlich Lipophilie, Hydrophilie und der Eignung zur Komplexierung von PGE2 darstellen, so dass sich diese Substanzen einerseits problemlos in wässriger Lösung formulieren lassen und andererseits in vivo in besonderem Maße die gewünschte Wirkung entfalten.

Die erfindungsgemäße Zusammensetzung kann insbesondere zur Administration i.v. (intravenös) oder i.pl. (intraplantar) formuliert sein. Es kann sich insbesondere um eine wässrige Lösung handeln.

Ein weiterer Gegenstand ist eine erfindungsgemäße Zusammensetzung, bei der das methylierte $\beta$-Cyclodextrin einen Substitutionsgrad von 9 bis 12, vorzugsweise 10 bis 12, weiter vorzugsweise 10 bis 11 aufweist, die erhältlich ist durch eine Fraktionierung, bevorzugt chromatografische Fraktionierung, von RAMEB.

Ein weiterer Gegenstand ist ein Verfahren zur Herstellung einer Zusammensetzung, bei der das methylierte $\beta$-Cyclodextrin einen Substitutionsgrad von 9 bis 12, vorzugsweise 10 bis 12, weiter vorzugsweise 10 bis 11 aufweist, mit den Schritten:

a. zur Verfügung stellen von RAMEB,

b. chromatografische Fraktionierung von RAMEB,

c. Abtrennen einer Fraktion oder mehrerer Fraktionen, so dass das erhaltene methylierte $\beta$-Cyclodextrin einen

Substitutionsgrad von 9 bis 12, vorzugsweise 10 bis 12, weiter vorzugsweise 10 bis 11 aufweist,

d. Formulierung einer pharmazeutischen Zusammensetzung mit dem in Schritt c. gewonnenen methyliertem β-Cyclodextrin als Wirkstoff.

Ausführungsbeispiele der Erfindung werden nachfolgend näher beschrieben. Die Zeichnungen werden im Kontext der jeweiligen Beispiele erläutert und näher beschrieben.

Beispiel 1

Chromatografische Fraktionierung von RAMEB

In diesem Beispiel wird RAMEB (Cavasol® W7 M Pharma, eingesetzte Menge 5 g) mittels Säulenchromatografie fraktioniert.

Säule:     d=20 mm, h=262 mm
Stationäre Phase: Merck Silicagel 60 (0,063-0,2 mm), 50 g
Mobile Phase:  Aceton und Methanol

Es wurden 3 Fraktionen gesammelt, die nachfolgend mit RAMEB-L, RAMEB-M und RAMEB-H (Low, Medium and High Lipophilicity) bezeichnet werden. Die Ausbeute an RAMEB-L betrug 32,2 Gew.-% des eingesetzten RAMEB.

Beispiel 2

Bestimmung des durchschnittlichen Substitutionsgrads der RAMEB-Fraktionen

10 mg der zu prüfenden Fraktion werden in ein 5 mm NMR-Röhrchen gegeben und 0,7 ml D2O zugegeben und gemischt. Anschließend wird ein 1H-NMR Spektrum zwischen 0 und 8 ppm aufgezeichnet (8 Scans, Aufzeichnung des FID für 4 s, 15 s Pause zwischen den Scans).
Die Flächen unter den Peaks zwischen 3,00 und 4,20 ppm (B, Methyl- und Glucopiranose-Protonen) und zwischen 4,95 und 5,41 ppm (A, glycosidische Protonen) werden integriert. Der durchschnittliche Substitutionsgrad errechnet sich nach folgender Formel:

$$DS = (B - 6A)/3$$

Die Ergebnisse sind wie folgt:

RAMEB-L: DS = 10,4
RAMEB-M: DS = 12
RAMEB-H: DS = 13

Beispiel 3

Bestimmung der Komplexstabilitätskonstanten RAMEB-Fraktionen/ PGE2

Die Komplexstabilitätskonstanten verschiedener RAMEB-Fraktionen mit PGE2 wurden in vitro durch Kapillarelektrophorese (CE) bestimmt. Die Versuchsbedingungen waren wie folgt:

Kapillare: 50/58,5 cm effektive Länge/Gesamtlänge (unfused silica)
Background-Elektrolyt (BGE): 30 mM Phosphatpuffer, pH=6,13 eingestellt mit TRIS
Injektion: 50 mbar, 4 s
Laufzeit: 10 min
Detektor: Diodenarray, direct mode, Sample-Wellenlänge 200/20 nm, Referenzwellenlänge 350/20 nm
Spannung: +30 kV
Kapillartemperatur: 25°C

Vorkonditionierung: 1 min Wasser, 1 min 0,1 N NaOH, 1 min Wasser, 4 min BGE

Probenvorbereitung:

PGE2-Stammlösung: 1 mg festes PGE2 (Sanofi Aventis) wird in einer 50:50 v/v-Mischung von EtOH und MQ-Wasser (Milli-Q purified water, Millipore) in einer Konzentration von 1 mg/ml gelöst. Die Stammlösung wurde tiefgefroren aufbewahrt.

PGE2-Probenlösung: PGE2-Stammlösung wurde 20fach mit MQ-Wasser verdünnt und mit 0,01 v/v% DMSO als EOF Marker versetzt (DMSO spiked).

Referenz: MQ-Wasser wurde mit 0,01 v/v% DMSO als EOF Marker versetzt (DMSO spiked).

Versuchsdurchführung:

Zunächst werden die Migrationszeiten von EOF (DMSO) und PGE2 bestimmt. Die jeweiligen Mobilitäten ($\mu$) werden wie folgt bestimmt:

$$\mu = (l_{eff} * l_{total})/(t_{mig} * U)$$

mit

$l_{eff}$ :   effektive Kapillarlänge
$l_{total}$ :   Gesamtkapillarlänge
$t_{mig}$ :   Migrationszeit [min]
$U$ :   Spannung [V]

Die effektive Mobilität wird wie folgt berechnet:

$$\mu_{eff} = \mu_{EOF} - \mu_{PGE2}$$

Diese Messungen wurden durchgeführt für PGE2-Probenlösung sowie für PGE2-Probenlösungen versetzt mit drei unterschiedlichen Konzentrationen (5, 10 und 15 mM) der zu untersuchenden RAMEB-Fraktion.

Mobilitätsdifferenzen wurden ermittelt durch Subtraktion des für die reine PGE2-Probenlösung ermittelten $\mu_{eff}$-Wertes von den $\mu_{eff}$-Werten, die bei der jeweiligen RAMEB-Konzentration gemessen wurden. Die ermittelten Mobilitätsdifferenzen (x-Achse) werden gegen den Quotienten aus der jeweiligen Mobilitätsdifferenz geteilt durch die jeweilige Konzentration der RAMEB-Fraktion (y-Achse) aufgetragen und eine Gerade durch diese Punkte gelegt. Die negative Steigung dieser Geraden ist die Komplexstabilitätskonstante (K). Folgende Werte wurden ermittelt:

| CD | DS | K [1/M] |
|---|---|---|
| RAMEB-L | 10,5 | 582 $\pm$ 6 |
| RAMEB-M | 12 | 450 $\pm$ 15 |
| RAMEB-H | 13 | 370 $\pm$ 20 |

Beispiel 4

In vivo Versuche an Wistar-Ratten

Alle Untersuchungen an männlichen Wistar Ratten zur Wirkung der selektierten Cyclodextrine wurden von der Regierung von Unterfranken genehmigt. Ratten wurden intraplantar (i.pl.) mit PGE2 oder CFA (Freund's Complete Adjuvant) injiziert. Nach 1 h bzw. 4 d wurden RAMEB oder RAMEB Fraktionen lokal (i.pl.) oder systemisch (i.v.) injiziert und mechanische (Randall-Sellito-Test) sowie thermische (Hargreaves Test) nozizeptive Schwellen gemessen bzw. Pfotengewebe für $PGE_2$ oder $PGD_2$ ELISA entnommen.

*CFA-induzierte Entzündung der Rattenhinterpfote:* CFA wird in einer Dosis von 150 $\mu$l in die rechte hintere Rattenpfote unter Isoflurannarkose induziert. Die Entzündung und der darauffolgende Schmerz in der entzündeten Pfote entwickeln sich innerhalb 2 bis 96 h. Bis zu diesem Zeitpunkt gibt es keinen signifikanten Unterschied im Essverhalten, Körpergewicht, Kerntemperatur oder dem generellen Aktivitätslevel im Vergleich zu den nicht behandelten Tieren (Stein C, Millan MJ, Shippenberg TS, Herz A. Neurosci Lett. 1988 Jan 22; 84(2):225-8.).

*PGE2-induzierte Hyperalgesie:* Ratten werden intraplantar (100 $\mu$l) mit unterschiedlichen Dosierungen von PGE2 unter Isoflurannarkose injiziert. Die Zeitabhängigkeit der Hyperalgesie wird nach 30 min bis 12 h untersucht. Der darauffolgende Schmerz in der ipsilateralen Pfote entwickelt sich innerhalb von 3 h (Ferreira SH, Molina N, Vettore O. Prostaglandins. 1982 Jan;23(1):53-60; Sachs D, Villarreal C, Cunha F, Parada C, Ferreira Sh. Br J Pharmacol. 2009 Mar;156(5):826-34. doi: 10.1111/j.1476-5381.2008.00093.x. Epub 2009 Feb 13). Die Tiere werden im gesamten Verlauf genau auf ihren aktuellen Gesundheitszustand hin untersucht (Aussehen, Verhalten). Nach dem Experiment werden die Ratten getötet.

*RAMEB oder Parecoxib:* Ratten werden intraplantar (100 $\mu$l) mit unterschiedlichen Dosierungen von RAMEB oder Parecoxib zusammen mit PGE2 oder 2 bzw. 96 h nach CFA von PGE2 unter Isoflurannarkose injiziert.

*Algesiometrische Messungen:* Dafür werden die Tiere wie folgt an die entsprechende Versuchsbedingungen von Tag 1-3 gewöhnt: Für die Gewöhnung für den Randall-Sellito-Test werden die Tiere 3 x pro Tag locker unter einem Zellstoff gehalten, um sie an die Situation des Experimentes zu gewöhnen. Für die Gewöhnung an den Hargreaves Test werden die Ratten täglich in die Plexiglaskammer gesetzt, um diese an die Situation des Experimentes zu gewöhnen.

Tag 4 Behandlung:

Intraplantare Injektionen werden unter einer Isoflurannarkose durchgeführt. Zehn Minuten nach der letzten Injektion erfolgt die Messung der Pfotendruckschwelle mittels Randall-Sellito-Test (Gerät von Ugo Basile) oder die Messung der Pfotenlatenz nach Hargreaves. Jede Pfote ipsilateral und kontralateral wird drei Mal im Abstand mindestens 10 s gemessen. Für den Randall-Sellito-Test wird die Ratte locker unter Zellstoff gehalten und die Pfote auf ein kleines Podest gesetzt. Dann wird von oben über einen Stempel ein steigender Druck auf die Pfote ausgeübt bis die Ratte die Pfote wegzieht (Stein C, Gramsch C, Herz A. J Neurosci. 1990 Apr; 10(4):1292-8; Rittner HL, Mousa SA, Labuz D, Beschmann K, Schäfer M, Stein C, Brack A. J Leukoc Biol. 2006 May;79(5):1022-32. Epub 2006 Mar 7).

Zur Messung der thermischen Hyperalgesie werden die Ratten in die durchsichtige Plastikkammer mit einem Glasboden (Model 336 Analgesia Meter, IITC Life Science, Woodland Hills, CA, USA) gesetzt, in dem sie sich frei bewegen können. Dann wird Wärme unter die plantare Pfotenfläche durch den Glasboden appliziert. Die Wärme wird mit einer hochintensiven Projektorlampe erzeugt. Dabei wird die Zeit mit einer elektronischen Stoppuhr gemessen, bis die Pfote weggezogen wird. Es werden zwei Messungen im Abstand von 30 s durchgeführt und der Mittelwert berechnet. Die Wärmeintensität wird so angepasst, dass der basale Wert 20 s beträgt und der Test nach 25 s beendet wird, um Gewebeschäden zu vermeiden (modifiziert nach Hargreaves K, Dubner R, Brown F, Flores C, Joris J. Pain. 1988 Jan;32(1):77-88).

Die Versuchsergebnisse werden nachfolgend unter Bezugnahme auf die Figuren erläutert.

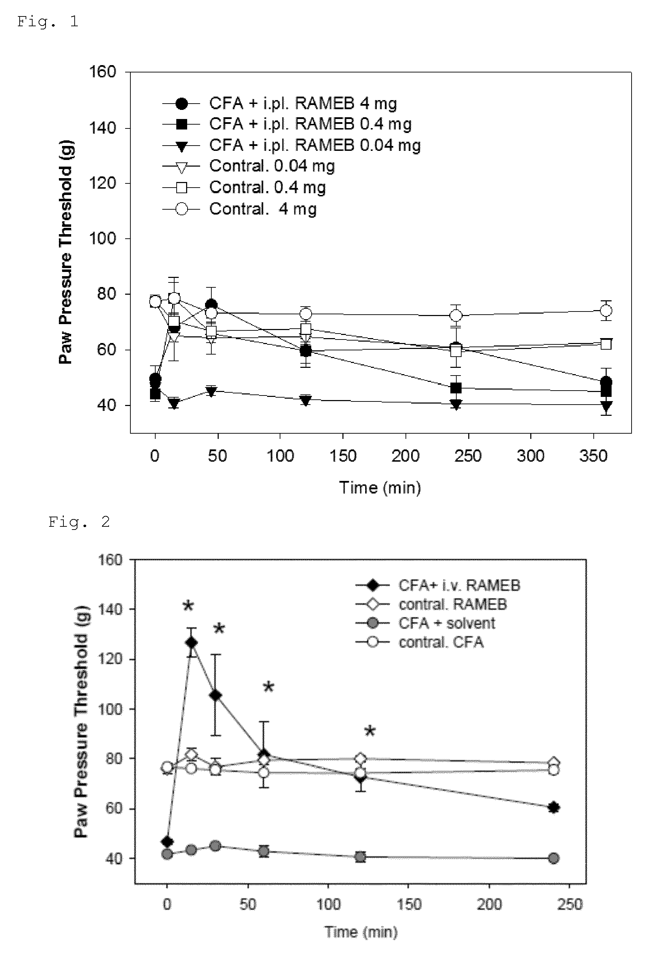

Fig. 1: Wistar-Ratten wurden i.pl. mit 0,15 ml CFA über 4 d injiziert zur Erzeugung einer lokalen entzündlichen Reaktion. Anschließend wurden die Ratten mit unterschiedlichen Dosierungen RAMEB, 0,04 mg (schwarze Dreiecke), 0,4 mg (schwarze Quadrate) und 4 mg (schwarze Kreise) behandelt und die nociceptive Pfotendruckschwelle bestimmt. Der Zeitpunkt Null zeigt die Basislinie vor der RAMEB-Behandlung. Die dosisabhängige Erhöhung des Pfotendruckschwellwerts (Paw Pressure Threshold) war maximal 45 min post Injektion in entzündeten Pfoten. Die kontralaterale (Contral.) Seite (weiße Symbole) zeigte keine wesentlichen Veränderungen.

Fig. 2: Wistar-Ratten wurden i.pl. mit 0,15 ml CFA über 4 d injiziert zur Erzeugung einer lokalen entzündlichen Reaktion. Anschließend wurden die Ratten mit 20 mg RAMEB i.v. (schwarze Diamanten) behandelt. Eine Kontrollgruppe (graue Kreise) wurde mit Lösungsmittel behandelt. Die nociceptive Pfotendruckschwelle wurde bestimmt. RAMEB i.v. zeigte im Vergleich zu RAMEB i.pl. einen ausgeprägteren Effekt mit einer ähnlichen Kinetik. Die kontralaterale Seite (weiße Symbole) zeigte keine wesentlichen Veränderungen.

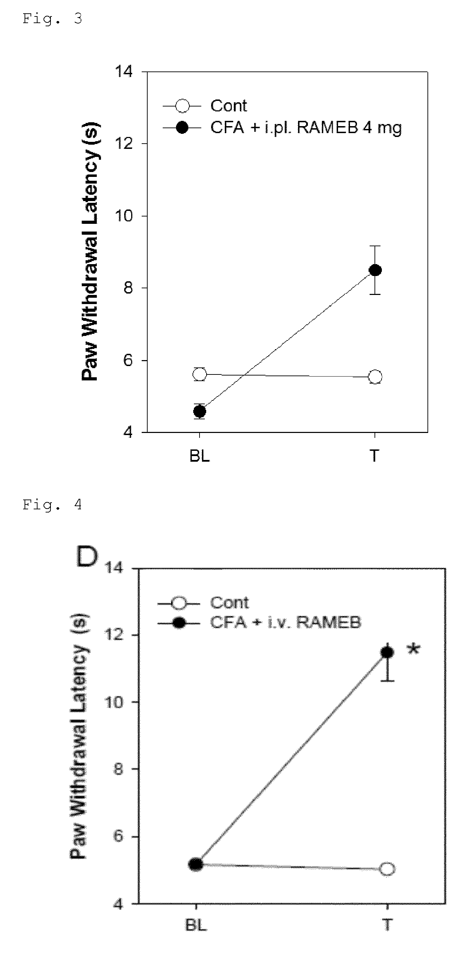

Fig. 3: Wistar-Ratten wurden i.pl. mit 0,15 ml CFA über 4 d injiziert zur Erzeugung einer lokalen entzündlichen Reaktion. Anschließend wurden die Ratten mit 4 mg RAMEB i.pl. (schwarze Kreise) behandelt. Eine Kontrollgruppe (weiße Kreise) wurde mit Lösungsmittel behandelt. Thermische nociceptive Schwellwerte (Zeit bis zum Wegziehen der Pfote in s) wurden vor (BL) und 15 und 45 min nach (T) RAMEB-Administration bestimmt (n=6, Two Way ANOVA, Bonferroni post hoc Korrektur, *P<0,05).

Fig. 4: Es wurde wie in Fig. 3 vorgegangen, jedoch mit 20 mg RAMEB i.v. behandelt.

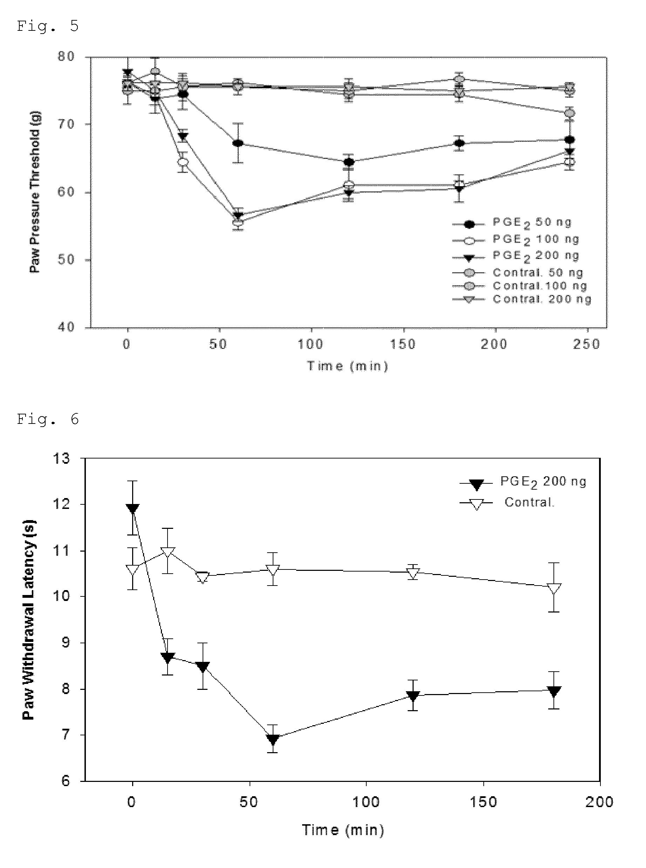

Fig. 5: Wistar-Ratten wurden i.pl. unterschiedliche Dosen PGE2 injiziert: 50 ng (schwarze Kreise), 100 ng (weiße Kreise) und 200 ng (schwarze Dreiecke). Pfotendruckschwellwerts wurden zu unterschiedlichen Zeitpunkten post i.pl. gemessen. Die dosisabhängige Absenkung des Schwellwertes war nach 60 min maximal. Die kontralaterale Seite (graue Symbole) zeigte keine wesentlichen Veränderungen.

Fig. 6: Wistar-Ratten wurden i.pl. 200 ng PGE2 injiziert (schwarze Dreiecke). Thermische nociceptive Schwellwerte wurden gemessen und mit unbehandelten kontralateralen Pfoten (weiße Dreiecke) verglichen.

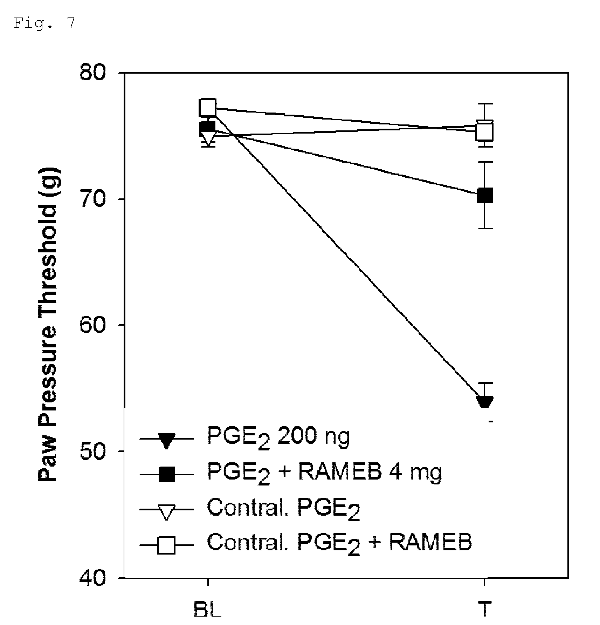

Fig. 7: Wistar-Ratten wurden i.pl. 200 ng PGE2 injiziert (schwarze Dreiecke). Einer weiteren Gruppe wurde 60 min nach Injektion von PGE2 4 mg RAMEB i.pl. injiziert (schwarze Quadrate). Pfotendruckschwellwerts wurden vor (BL) und 15 und 45 min nach (T) RAMEB-Administration bestimmt ($n=6$, Two Way ANOVA, Bonferroni post hoc Korrektur, *$P<0,05$). Als Vergleich wurden kontralaterale unbehandelte Pfoten herangezogen (weiße Dreiecke und Quadrate).

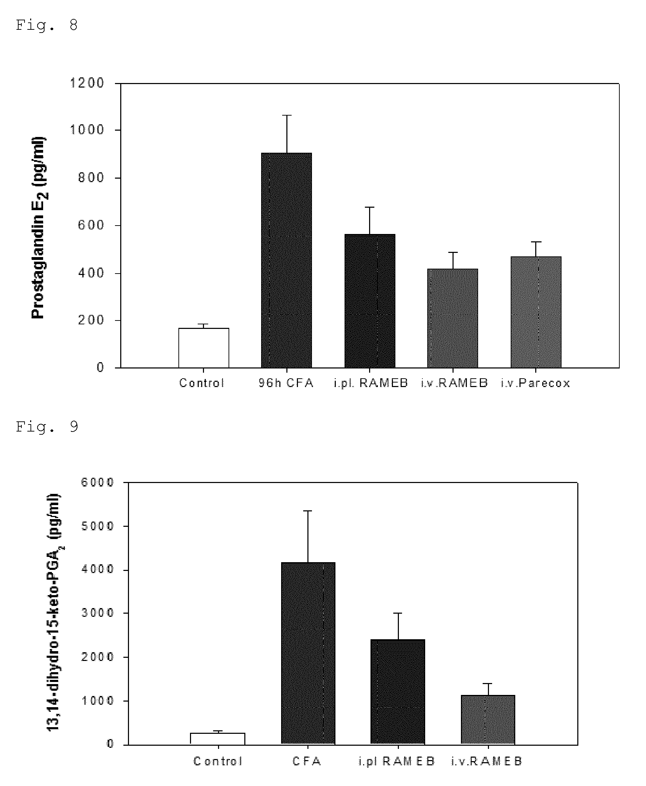

Fig. 8 und 9: Gruppe 1 (linker erster Balken): Eine Kontrollgruppe von Ratten blieb unbehandelt. Gruppen 2 bis 5 (Balken 2 bis 5): Wistar-Ratten wurden i.pl. mit 0,15 ml CFA über 4 d injiziert zur Erzeugung einer lokalen entzündlichen Reaktion. Gruppe 2: Keine weitere Behandlung. Gruppen 3 bis 5: Anschließend wurden die Ratten mit 4 mg RAMEB i.pl. (Gruppe 3), 8 mg RAMEB i.v. (Gruppe 3) bzw. 5 mg Parecoxib (Gruppe 5, selektiver COX2-Hemmer, Vergleichsversuch) behandelt. Pfotengewebe wurde entnommen (Gruppe 2 15 min, Gruppe 3 45 min und Gruppe 2 30 min nach RAMEB bzw. Parecoxib-Administration) und mittels ELISA auf PGE2 (Fig. 8) und PGE2-Metaboliten (13,14-Dihydro-15-keto-Prostaglandin A2, Fig. 9) untersucht($n=5$, ANOVA, Bonferroni post hoc Korrektur, $P<0,05$). Das Beispiel belegt die in vivo Wirkung von RAMEB auf PGE2.

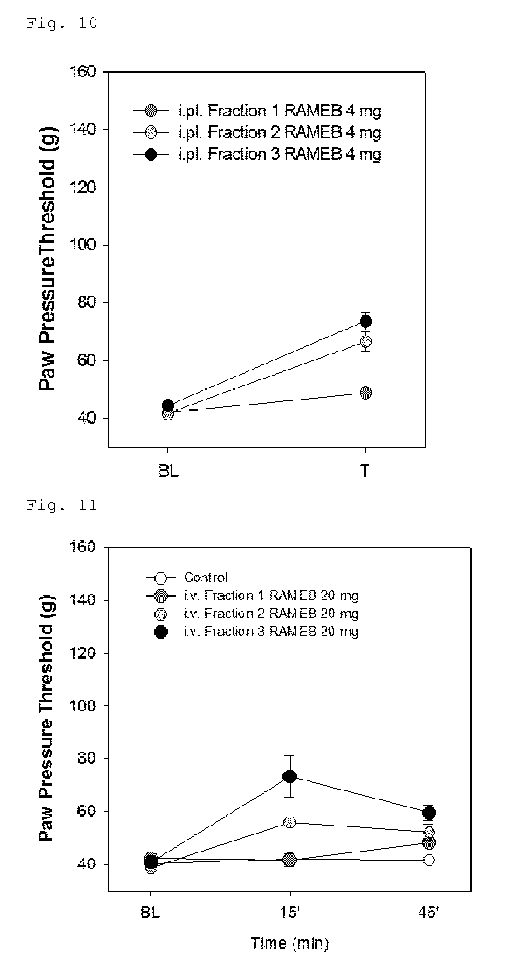

Fig. 10: Wistar-Ratten wurden i.pl. mit 0,15 ml CFA über 4 d injiziert zur Erzeugung einer lokalen entzündlichen Reaktion. Anschließend wurden die Ratten mit unterschiedlichen RAMEB-Fraktionen (4 mg i.pl.), RAMEB-H (dunkelgraue Kreise), RAMEB-M (graue Kreise) und RAMEB-L (schwarze Kreise) behandelt und nach 45 min (Zeitpunkt T) die nociceptive Pfotendruckschwelle bestimmt. Der Zeitpunkt Null (BL) zeigt die Basislinie vor der RAMEB-Behandlung.

Fig. 11: Wistar-Ratten wurden i.pl. mit 0,15 ml CFA über 4 d injiziert zur Erzeugung einer lokalen entzündlichen Reaktion. Anschließend wurden die Ratten mit unterschiedlichen RAMEB-Fraktionen (20 mg i.v.), RAMEB-H (dunkelgraue Kreise), RAMEB-M (graue Kreise) und RAMEB-L (schwarze Kreise) behandelt und nach 15 und 45 min die nociceptive Pfotendruckschwelle bestimmt. Der Zeitpunkt Null (BL) zeigt die Basislinie vor der RAMEB-Behandlung. Die Kontrollgruppe (weiße Kreise) wurde lediglich mit Lösungsmittel behandelt. In den Fig. 10 und 11 erkennt man, dass unter den untersuchten Fraktionen RAMEB-L die beste Wirkung hat.

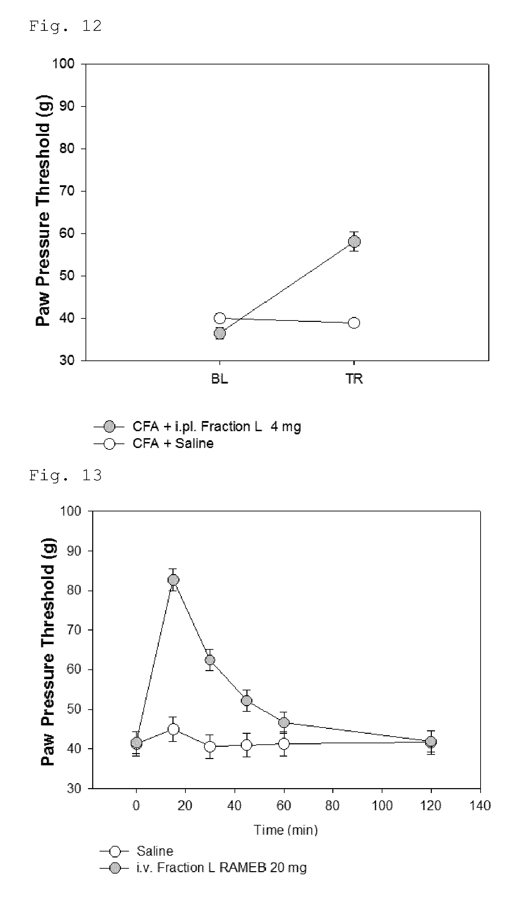

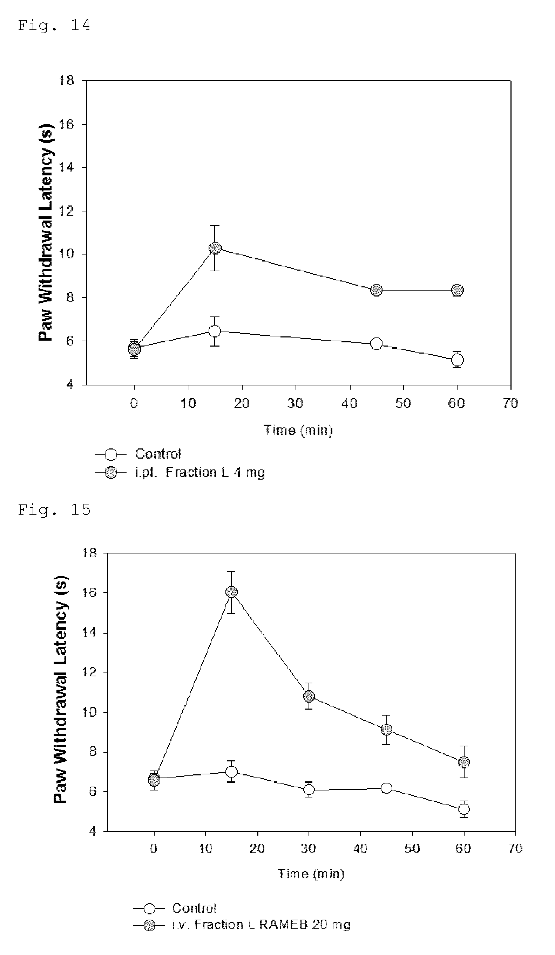

Fig. 12 bis 15: Wistar-Ratten wurden i.pl. mit 0,15 ml CFA über 4 d injiziert zur Erzeugung einer lokalen entzündlichen Reaktion. Anschließend wurden die Ratten mit RAMEB-L (Fig. 13 und 15:20 mg i.v., Fig. 12 und 14: 4 mg i.pl.) behandelt. Die nociceptive Pfotendruckschwelle (Fig. 12 und 13) sowie thermische nociceptive Schwellwerte (Fig. 14 und 15) wurden im Zeitpunkt Null (BL) sowie nach den gezeigten Zeitabständen (T=45 min) bestimmt (graue Kreise, $n=6$, Two Way ANOVA, Bonferroni post hoc Korrektur, *$P<0,05$). Als Vergleich wurden mit Lösungsmittel behandelte kontralaterale Pfoten herangezogen (weiße Kreise). Man erkennt, dass RAMEB-L sowohl bei i.v. als auch i.pl. Gabe inflammatorische thermische Hyperalgesie reversiert und mechanische Hyperalgesie abschwächt.

**Patentansprüche**

1. Pharmazeutische Zusammensetzung enthaltend methyliertes β-Cyclodextrin zur Verwendung als Analgetikum und/oder Antiphlogistikum.

2. Zusammensetzung nach Anspruch 1, **dadurch gekennzeichnet, dass** das methylierte β-Cyclodextrin RAMEB ist.

3. Zusammensetzung nach Anspruch 1, **dadurch gekennzeichnet, dass** das methylierte β-Cyclodextrin einen Substitutionsgrad von 9 bis 12, vorzugsweise 10 bis 12, weiter vorzugsweise 10 bis 11 aufweist.

4. Zusammensetzung nach einem der Ansprüche 1 bis 3, **dadurch gekennzeichnet, dass** sie zur Administration i.v. oder i.pl. formuliert ist.

**5.** Zusammensetzung nach einem der Ansprüche 1 bis 4, **dadurch gekennzeichnet, dass** sie als wässrige Lösung formuliert ist.

**6.** Zusammensetzung nach einem der Ansprüche 1 bis 5, **dadurch gekennzeichnet** zur Verwendung als Analgetikum bei entzündlichen Erkrankungen, an denen PGE2 beteiligt ist.

**7.** Zusammensetzung nach Anspruch 3, erhältlich durch eine chromatografische Fraktionierung von RAMEB.

**8.** Verfahren zur Herstellung einer Zusammensetzung nach Anspruch 3, mit den Schritten:

a. zur Verfügung stellen von RAMEB,
b. chromatografische Fraktionierung von RAMEB,
c. Abtrennen einer Fraktion oder mehrerer Fraktionen, so dass das erhaltene methylierte β-Cyclodextrin einen Substitutionsgrad von 9 bis 12, vorzugsweise 10 bis 12, weiter vorzugsweise 10 bis 11 aufweist,
d. Formulierung einer pharmazeutischen Zusammensetzung mit dem in Schritt c. gewonnenen methyliertem β-Cyclodextrin als Wirkstoff.

Fig. 1

Fig. 2

Fig. 3

Fig. 4

Fig. 5

Fig. 6

Fig. 7

Fig. 8

Fig. 9

Fig. 10

Fig. 11

Fig. 12

—●— CFA + i.pl. Fraction L  4 mg
—○— CFA + Saline

Fig. 13

—○— Saline
—●— i.v. Fraction L RAMEB 20 mg

Fig. 14

Fig. 15

**Europäisches Patentamt**
**European Patent Office**
**Office européen des brevets**

## EUROPÄISCHER RECHERCHENBERICHT

Nummer der Anmeldung

EP 14 15 5234

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Reine-Solange Sauer ET AL: "Hemmung der Wirkung von Prostaglandin E2 durch Komplexierung mit methylierten Beta-Cyclodextrinen zur Behandlung von entzündungsbedingten Schmerzen in Ratten", , 22. Januar 2014 (2014-01-22), XP055106379, Gefunden im Internet: URL:http://www.anaesthesie.ukw.de/uploads/media/Sauer_Wuerzburg.pdf [gefunden am 2014-03-10] * Zusammenfassung * ----- | 1-8 | INV. A61K31/724 A61K9/08 A61K9/00 A61P29/00 |
| X | WO 97/17977 A1 (EVEREST TODD RESEARCH & DEV LI [GB]; TODD SELWYN EVEREST [GB]) 22. Mai 1997 (1997-05-22) * das ganze Dokument * * Seite 2, Zeile 1 - Zeile 5 * * Beispiele I,III,VI,VII * * Ansprüche 1-3 * ----- | 1-6 | |
| | | | RECHERCHIERTE SACHGEBIETE (IPC) A61K A61P |

Der vorliegende Recherchenbericht wurde für alle Patentansprüche erstellt

| Recherchenort | Abschlußdatum der Recherche | Prüfer |
|---|---|---|
| München | 11. März 2014 | Economou, Dimitrios |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 14 15 5234

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-03-2014

| Im Recherchenbericht angeführtes Patentdokument | Datum der Veröffentlichung | Mitglied(er) der Patentfamilie | Datum der Veröffentlichung |
|---|---|---|---|
| WO 9717977 A1 | 22-05-1997 | AU 719360 B2 | 04-05-2000 |
| | | AU 7578796 A | 05-06-1997 |
| | | CA 2237758 A1 | 22-05-1997 |
| | | EP 0863761 A1 | 16-09-1998 |
| | | GB 2307176 A | 21-05-1997 |
| | | NZ 322119 A | 24-09-1998 |
| | | WO 9717977 A1 | 22-05-1997 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **STEIN C ; MILLAN MJ ; SHIPPENBERG TS ; HERZ A.** *Neurosci Lett.,* 22. Januar 1988, vol. 84 (2), 225-8
- **FERREIRA SH ; MOLINA N ; VETTORE O.** *Prostaglandins,* Januar 1982, vol. 23 (1), 53-60
- **SACHS D ; VILLARREAL C ; CUNHA F ; PARADA C ; FERREIRA SH.** *Br J Pharmacol.,* Marz 2009, vol. 156 (5), 826-34
- **STEIN C ; GRAMSCH C ; HERZ A.** *J Neurosci.,* April 1990, vol. 10 (4), 1292-8
- **RITTNER HL ; MOUSA SA ; LABUZ D ; BESCHMANN K ; SCHÄFER M ; STEIN C ; BRACK A.** *J Leukoc Biol.,* 07. Marz 2006, vol. 79 (5), 1022-32
- **HARGREAVES K ; DUBNER R ; BROWN F ; FLORES C.** *Joris J. Pain.,* Januar 1988, vol. 32 (1), 77-88